Europäisches Patentamt

European Patent Office

Office européen des brevets

(11) Numéro de publication: **0 402 258**
**A1**

## (12) DEMANDE DE BREVET EUROPEEN

(21) Numéro de dépôt: **90401551.8**

(22) Date de dépôt: **07.06.90**

(51) Int. Cl.5: **A63K 3/00, A01K 15/02, A61B 5/11, G01P 1/07**

(30) Priorité: **09.06.89 FR 8907641**

(43) Date de publication de la demande:
**12.12.90 Bulletin 90/50**

(84) Etats contractants désignés:
**AT BE CH DE ES GB IT LI LU NL SE**

(71) Demandeur: **ASSOCIATION PERSIVAL**
**Ecole Nationale d'Equitation**
**F-49400 Saumur(FR)**

(72) Inventeur: **Le Nouvel, Alain**
**35, rue Neuve**
**F-78610 Les Brevières(FR)**
Inventeur: **Dronka, Thierry**
**1, rue Picabia**
**F-94000 Creteil(FR)**
Inventeur: **Maginot, André**
**7, rue Léonie**
**F-94500 Champigny-sur-Marne(FR)**
Inventeur: **Daveine, Michel**
**28, rue des Trois Bornes**
**F-75011 Paris(FR)**
Inventeur: **Jouffroy, Jean-Louis**
**172, rue de Grenelle**
**F-75007 Paris(FR)**

(74) Mandataire: **Loyer, Bertrand et al**
**Cabinet Pierre Loyer 77, rue Boissière**
**F-75116 Paris(FR)**

(54) **Dispositif de prise des mesures des divers éléments constitutifs du mouvement d'un mobile.**

(57) Dispositif de prises des mesures des divers éléments constitutifs du mouvement d'un mobile, caractérisé par le fait qu'il est constitué par un support (1) ayant une forme d'arceau à deux branches de façon à pouvoir enjamber par exemple le garrot (19) d'un cheval, ledit support (1) comportant un point haut et deux points bas, symétriques par rapport au plan longitudinal médian dudit support (1) ; ledit support (1) portant six accéléromètres (3, 4, 5, 9, 10, 13) dont trois (3, 4, 5) sont disposés de façon que leurs axes (6, 7, 8) soient parallèles à l'axe longitudinal X, deux (9, 10) sont disposés de façon que leurs axes (11, 12) soient parallèles à l'axe vertical Z et un accéléromètre (13) disposé de façon que son axe (14) soit parallèle à l'axe transversal Y.

Fig. 1

Dans la demande de brevet français n° 87.08050 déposée le 10 juin 1987 et publiée sous le n° 2.616.337, on a décrit un procédé permettant de tracer les courbes figuratives des variations des différentes composantes du mouvement d'un cheval et de les restituer dans un simulateur.

Selon ce procédé, on place sur un cheval en mouvement réel des moyens de mesure tels que des accéléromètres et des gyromètres, ou encore une centrale inertielle par l'intermédiaire desquels on mesure les vitesses de déplacement linéaire selon les trois axes X, Y et Z ainsi que les déplacements en rotation selon ces axes.

Pour mettre en oeuvre ce procédé, on a dans un premier temps arrimé une centrale à inertie, du type de celles qui sont employées dans les avions, sur la sel le d'un cheval et on a accouplé à cette centrale à inertie un dispositif d'enregistrement magnétique. Un tel équipement s'est rapidement révélé peu satisfaisant. En effet, un tel matériel relativement lourd et encombrant exclut la présence d'un cavalier de sorte que le cheval est livré à lui-même et que l'on est obligé de le mettre à la longe pour enregistrer les diverses allures (pas, trot, galop, saut) et que celles-ci ne donnent donc que des indications relatives à un parcours circulaire ou sur de courtes lignes droites. En outre, le centre de gravité d'un tel équipement, forcément assez volumineux se trouve situé trop haut au-dessus du dos du cheval ce qui fausse les mesures d'accélérations et oblige à introduire des calculs de correction d'erreur qui ne sont que des approximations. Enfin, du fait que cet équipement est lourd et fixé au-dessus de la selle, il est difficile d'empêcher qu'il ne soit soumis a des mouvements parasites dus au ballant, ce qui, là encore, fausse les mesures et est très complexe à corriger.

On a donc proposé d'employer une sursel le métallique, c'est-à-dire une pièce métallique conformée de façon qu'elle épouse exactement la forme d'une selle classique en cuir; puis on a fixé très solidement cette sursel le à la selle ; enfin on a soudé sur cette sursel le une plate-forme métallique portant trois gyromètres, trois accéléromètres.

On a ainsi réussi à éliminer pratiquement tous les mouvements parasites des moyens de mesure ; mais, bien que le centre de gravité de cet équipement ait été abaissé, il se trouvait encore une douzaine de cm environ au-dessus du dos du cheval et, surtout comme précédemment le cheval se trouvait toujours sans cavalier.

La présente invention concerne un dispositif grâce auquel on est parvenu à éliminer tous les inconvénients précités.

Ce dispositif est constitué par une pièce de support ayant une forme arquée à deux branches de façon à comporter un point haut médian et deux points bas symétriques par rapport au plan médian du support, la partie intérieure de cette pièce arquée ayant un profil analogue à la section du garrot d'un cheval , ce support portant six accéléromètres, trois disposés selon des axes parallèles à l'axe longitudinal X, deux disposés selon des axes parallèles à l'axe vertical Z et un disposé selon l'axe transversal Y.

De préférence, ce support est fixé à l'arçon de la selle immédiatement devant ledit arçon de façon que le point de convergence O de l'axe de symétrie du support et de l'axe transversal Y soit situé au niveau du garrot du cheval.

On obtient ainsi un équipement très léger, inférieur à 2 kg; ne comportant pratiquement aucun décalage des moyens de mesure par rapport au garrot du cheval ; et permettant au cheval d'être monté par son cavalier qui, lui, porte sur son dos le système d'enregistrement.

A titre d'exemple non limitatif , on a représenté aux dessins annexés un mode de réalisation de la présente invention.

La figure 1 est une vue de face du support ;

La figure 2 est une vue en perspective du support selon l'invention monté sur une selle.

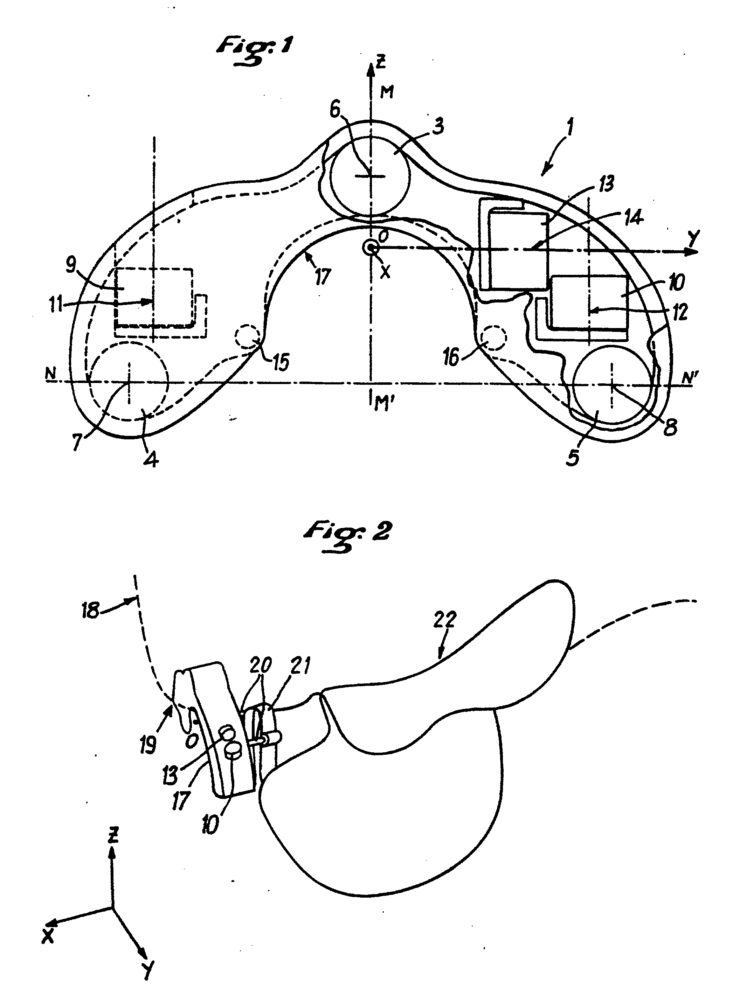

En se reportant à la figure 1, on voit que le dispositif comporte un support ou boîtier 1 ayant une forme générale en arceau, ou en V arrondi inversé de façon à comporter un point haut situé sur le plan de symétrie MM' et deux points bas, symétriques par rapport audit plan MM'.

Au point haut est placé un accéléromètre 3 dont l'axe 6 est parallèle à l'axe X longitudinal. Aux points bas, sont placés deux accéléromètres 4 et 5 dont les axes respectifs 7 et 8 sont, également, parallèles audit axe X et situés dans un plan NN' horizontal et perpendiculaire au plan MM'.

Au voisinage des accéléromètres 4 et 5 sont disposés deux autres accéléromètres 9 et 10. Ces deux accéléromètres sont symétriques par rapport au plan de symétrie MM'. Et d'autre part, leurs axes respectifs 11 et 12 sont parallèles à l'axe Z vertical.

Sur l'une des deux branches du support 1 est disposé un sixième et dernier accéléromètre 13 dont l'axe 14 est parallèle à l'axe Y horizontal transverse, perpendiculaire au plan de symétrie MM'.

Dans l'exemple représenté, les six accéléromètres 3, 4, 5, 9, 10, 13 sont dans des boîtiers cylindriques ayant environ 3 cm de diamètre et 3 cm de hauteur.

Le support comporte en outre deux orifices de fixation 15 et 16, symétriques par rapport au plan MM', qui, de préférence sont légèrement coniques et destiné à recevoir deux doigts de fixation 20 portés par une pièce métallique 21 solidaire de l'arçon d'une selle 22 (figure 2), le dispositif permet le démontage rapide et le remontage en position identique par rapport à la selle.

Sur la figure 2, on a représenté de façon schématique par un trait tireté 18 le corps du cheval portant la selle 22, le garrot étant indiqué en 19. On voit que le contour intérieur 17 du support 1 est déterminé de façon à enjamber en quelque sorte le garrot 19 du cheval et on voit que l'axe 14 de l'accéléromètre 13 coupe en un point O le plan de symétrie MM', la forme du support 1 et l'emplacement dudit axe 14 étant déterminés de façon que ledit point O soit, comme représenté aux figures 1 et 2, au niveau du garrot 19 du cheval. L'orientation des axes X et Z peut être inclinée dans le plan de symétrie MM' pour dégager l'encolure et s'adapter à la selle.

Le fonctionnement du dispositif ainsi décrit est le suivant.

Les déplacements linéaires sont mesurés : selon l'axe X par les trois accéléromètres 3, 4 et 5, on calcule l'accélération linéaire au point 0, aux moyens de coefficients résultants de la géométrie du montage à partir de mesures des accéléromètres 3,4 et 5. Selon l'axe Z, on obtient l'accélération au point O par la moyenne des mesures des deux accéléromètres 9 et 10 et selon l'axe Y par la mesure fournie par l'accéléromètre 13.

Pour les rotations, on ne dispose pas de gyromètres mais uniquement des accéléromètres. La mesure de la rotation autour de l'axe Z ou axe de lacet est obtenue par le différentiel entre les deux accéléromètres 4 et 5 ; la mesure de la rotation autour de l'axe Y ou axe de tangage est obtenue par le différentiel entre, d'une part la mesure provenant de l'accéléromètre 3 et d'autre part la moyenne des mesures provenant des deux accéléromètres 4 et 5 ; la mesure de la rotation autour de l'axe Y ou axe de roulis est donné par le différentiel entre les mesures fournies par les deux accéléromètres 9 et 10, ces calculs fournissant les accélérations angulaires.

On obtient ainsi des mesures d'une précision excellente alors que cela n'était pas possible avec les dispositifs connus du fait de la multiplicité des calculs correctifs qu'il fallait introduire. Les mesures sont réalisées avec des moyens beaucoup moins encombrants, beaucoup plus légers et aussi beaucoup plus simples et fiables puisse qu'il n'est plus besoin d'avoir des gyromètres. Enfin ce dispositif autorise la présence d'un cavalier sur la selle 22 et donc de diriger à volonté le cheval et de lui faire exécuter les différentes allures et figures dans des conditions naturelles.

Dans l'exemple qui a été réalisé à fins d'expérimentation, la largeur du support 1 était de 30 cm, sa hauteur de 16 cm, son épaisseur de 8 cm et son poids de 2 kg. Les différents accéléromètres 3, 4, 5, 9, 10 et 13 sont reliés à une prise à plots multiples , qui n'est pas représentée et se trouve au bout d'un câble multiconducteur d'une longueur d'une vingtaine de centimètres environ. Le cavalier porte sur son dos les moyens d'enregistrement qui sont reliés à ladite prise. Le cavalier ou le cheval porte les batteries d'alimentation électrique. La charge portée par le cavalier était de l'ordre de 8 kg.

Bien entendu sans sortir du cadre de l'invention, les moyens d'enregistrement et l'alimentation pourront être portés par une personne se déplaçant à côté du cheval.

La présente invention a été décrite en relation avec l'étude du mouvement d'un cheval, le dispositif étant fixé à l'arçon d'une selle. Il doit cependant être bien compris qu'il ne s'agit là que d'un exemple de réalisation qui n'est pas limitatif. En effet, ce dispositif peut être employé pour la prise de mesures des divers éléments constitutifs du mouvement de tout mobile quel qu'il soit.

## Revendications

1. Dispositif de prises des mesures des divers éléments constitutifs du mouvement d'un mobile , caractérisé par le fait qu'il est constitué par un support (1) ayant une forme d'arceau à deux branches de façon à pouvoir enjamber par exemple le garrot (19) d'un cheval, ledit support (1) comportant un point haut et deux points bas, symétriques par rapport au plan longitudinal médian dudit support (1) ; ledit support (1) portant six accéléromètres (3, 4, 5, 9, 10, 13) dont trois (3, 4, 5) sont disposés de façon que leurs axes (6, 7, 8) soient parallèles à l'axe longitudinal X, deux (9, 10) sont disposés de façon que leurs axes (11, 12) soient parallèles à l'axe vertical Z et un accéléromètre (13) disposé de façon que son axe (14) soit parallèle à l'axe transversal Y.

2. Dispositif selon la revendication 1, dans lequel les mesures de déplacements linéaires se font : selon l'axe X par moyenne des mesures provenant des trois accéléromètres (3, 4, 5) dont les axes (6, 7, 8) sont parallèles à l'axe X; selon l'axe Z par la moyenne des mesures provenant des deux accéléromètres (9, 10) dont les axes (11, 12) sont parallèles audit axe Z ; selon l'axe Y par la mesure provenant de l'accéléromètre (13) dont l'axe (14) est parallèle audit axe Y ; tandis que les mesures de déplacements en rotation se font : selon l'axe de lacet Z par le différentiel des mesures provenant des deux accéléromètres (4, 5); selon l'axe de tangage Y par le différentiel de mesure provenant d'une part de la moyenne des deux accéléromètres (4, 5) et de l'accéléromètre (3) ; selon l'axe de roulis X par le différentiel des mesures provenant des deux accéléromètres (9, 10).

3. Dispositif selon la revendication 2, dans lequel les mesures d'accélérations angulaires et linéaires effectuées selon trois axes trirectangles, peuvent être intégrées pour restituer les vitesses et déplacements angulaires et linéaires et donc l'ensemble du mouvement du mobile rapporté en un point.

4. Dispositif selon l'une quelconque des revendications précédentes, dans lequel le mobile est un cheval, la partie intérieure du support (1) ayant un profil (17) correspondant approximativement au contour (18) du corps d'un cheval au niveau du garrot (19) et le dispositif étant fixé à l'arçon de la selle (22) de façon que le point de convergence O de l'axe MM′ de symétrie du support (1) et de l'axe (14) de l'accéléromètre transveral (13) soit situé au niveau du garrot (19) du cheval.

5. Dispositif selon la revendication 4, caractérisé par le fait que le support (1) comporte deux orifices de fixation (15, 16) dans lesquels pénètrent deux doigts (20) portés par une pièce métallique (21) solidaire de l'arçon d'une selle (22), de préférence près de la sangle ou sur un surfaix (notamment pour les chevaux malades).

6. Dispositif selon les revendications 4 et 5, dans lesquels les accéléromètres (3, 4, 5, 9, 10, 13) sont connectés électriquement à une source d'énergie électrique et à des moyens d'enregistrement portés soit par le cavalier soit le cheval, soit par une personne se déplaçant à côté du cheval.

7. Dispositif selon l'une quelconque des revendications 4, 5 et 6, dans lequel l'ensemble du mouvement du cheval, est rapporté en un point situé au niveau du garrot.

*Fig. 1*

*Fig. 2*

Office européen
des brevets

RAPPORT DE RECHERCHE EUROPEENNE

Numero de la demande

EP  90 40 1551

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | EP-A-0 296 023 (ECOLE NATIONALE D'EQUITATION) * Colonne 3, lignes 10-18,45-51 * --- | 1 | A 63 K    3/00 A 01 K   15/02 A 61 B    5/11 G 01 P    1/07 |
| A | DE-A-3 306 813 (ZOTTNIK) * Page 13, lignes 1-16; figure 5 * --- | 1 | |
| A | US-A-3 955 562 (FARRAR) * Colonne 1, lignes 49-61; figure 3 * --- | 1 | |
| A | WO-A-8 101 507 (SVENSSON) * Page 2, ligne 20 - page 3, ligne 30 * ----- | 1 | |

**DOMAINES TECHNIQUES RECHERCHES (Int. Cl.5)**

A 63 B
A 63 K
A 01 K
G 01 P
A 61 B

Le présent rapport a été établi pour toutes les revendications

| Lieu de la recherche | Date d'achèvement de la recherche | Examinateur |
|---|---|---|
| LA HAYE | 06-09-1990 | SCHOENLEBEN J.E.F. |